(19) Europäisches Patentamt
European Patent Office
Office européen des brevets

(11) Veröffentlichungsnummer : **0 085 785**
**B1**

(12) # EUROPÄISCHE PATENTSCHRIFT

(45) Veröffentlichungstag der Patentschrift : **11.12.85**

(51) Int. Cl.[4] : **C 07 D237/16**, A 01 N 43/58

(21) Anmeldenummer : **82111951.8**

(22) Anmeldetag : **23.12.82**

(54) **Substituierte 4,5-Dimethoxy-pyridazone, Verfahren zu ihrer Herstellung und ihre Verwendung zur Bekämpfung unerwünschten Pflanzenwuchses.**

(30) Priorität : **28.01.82 DE 3202678**

(43) Veröffentlichungstag der Anmeldung : **17.08.83 Patentblatt 83/33**

(45) Bekanntmachung des Hinweises auf die Patenterteilung : **11.12.85 Patentblatt 85/50**

(84) Benannte Vertragsstaaten : **BE CH DE FR GB IT LI NL**

(56) Entgegenhaltungen :
**EP-A- 0 003 805**
**EP-A- 0 037 925**

(73) Patentinhaber : **BASF Aktiengesellschaft**
**Carl-Bosch-Strasse 38**
**D-6700 Ludwigshafen (DE)**

(72) Erfinder : **Parg, Adolf, Dr.**
**Paray-le-Monial-Strasse 8**
**D-6702 Bad Duerkheim (DE)**
Erfinder : **Wuerzer, Bruno, Dr.**
**Ruedigerstrasse 13**
**D-6701 Otterstadt (DE)**
Erfinder : **Hamprecht, Gerhard, DR.**
**Rote-Turm-Strasse 28**
**D-6940 Weinheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 085 785 B1

**Beschreibung**

Die Erfindung betrifft 4,5-Dimethoxy-pyridazone, Verfahren zu ihrer Herstellung, Herbizide, die diese Verbindungen als Wirkstoffe enthalten, sowie Verfahren zur Bekämpfung unerwünschten Pflanzenwuchses mit diesen Verbindungen.

Es ist bekannt, daß 1-Phenyl-4,5-dimethoxy-pyridazin-6-one (DE-PS 11 97 676 ; DE-OS 30 13 267) und 1-Phenyl-4-methoxy-5-halogen-pyridazin-6-one (DE-PS 16 70 315) herbizid wirksam sind.

Es wurde gefunden, daß substituierte 4,5-Dimethoxy-Pyridazone der Formel

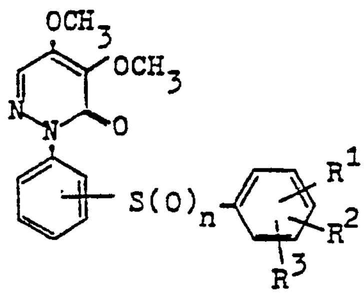

(I)

in der $R^1$, $R^2$ und $R^3$ jeweils unabhängig voneinander Wasserstoff, Halogen, Nitro, Cyano, Carboxyl, Alkyl, Halogenalkyl, Alkoxy, Halogenalkoxy, Alkylmercapto, Halogenalkylmercapto, Alkylsulfinyl oder Alkylsulfonyl mit jeweils 1 bis 4 Kohlenstoffatomen bedeuten und n für 0, 1 oder 2 steht,

eine gute herbizide Aktivität besitzen und dabei eine überraschende Verträglichkeit für Kulturpflanzen bzw. selektive herbizide Wirksamkeit bei Anwendung im Kulturpflanzenanbau aufweisen.

$R^1$, $R^2$ und $R^3$ können jeweils unabhängig voneinander beispielsweise Wasserstoff, Fluor, Chlor, Brom, Jod, Nitro, Cyano, Carboxyl, Methyl, Ethyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, n-Propyloxy, i-Propyloxy, tert.-Butyloxy, Trichlormethoxy, Trifluormethoxy, 1-Chlorethoxy, 2-Chlorethoxy, 1-Fluorethoxy, 2-Fluorethoxy, 2,2,2-Trichlorethoxy, 2,2,2-Trifluorethoxy, 1,1,2,2-Tetrafluorethoxy, 1,1,2,2,2-Pentafluorethoxy, Methylmercapto, Ethylmercapto, Trichlormethylmercapto, Trifluormethylmercapto, Methylsulfinyl, Ethylsulfinyl, Methylsulfonyl oder Ethylsulfonyl bedeuten, wobei $R^1$, $R^2$ und $R^3$ bevorzugt in 2,4,6-, 3,4,6- oder 3,4,5-Stellung zur Verknüpfungsstelle des Phenylrestes mit der Schwefelbrücke stehen.

Bevorzugte Verbindungen sind solche, bei denen

der Rest 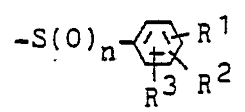

in 3- oder 4-Stellung am Phenylring steht und $R^1$, $R^2$ und $R^3$ Wasserstoff, Halogen oder Halogenalkyl mit 1 bis 4 Kohlenstoffatomen, bevorzugt Wasserstoff, Fluor, Chlor oder Trifluormethyl bedeuten.

Die Pyridazone der Formel I kann man durch Umsetzung eines 4,5-Dihalogenpyridazons der Formel

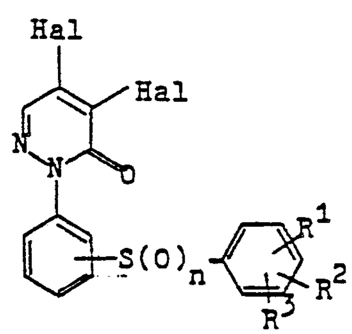

(II)

in der $R^1$, $R^2$, $R^3$ und n die obengenannten Bedeutungen haben und Hal Halogen, insbesondere Chlor oder Brom, bedeutet, mit ungefähr der stöchiometrischen Menge eines Alkalimethanolats in Gegenwart eines organischen Lösungsmittels bei einer Temperatur von im allgemeinen bis zu 100 °C erhalten, wobei man die Umsetzung drucklos oder unter Druck (1 bis 10 bar) kontinuierlich oder diskontinuierlich durchführt.

Verwendet man 1-[3'-(2''-Chlor-4''-trifluormethylphenyl-thio)-phenyl]-4,5-dichlor-pyridaz-6-on und

Natriummethylat als Ausgangsmaterialien, so kann der Reaktionsablauf durch folgendes Formelschema wiedergegeben werden :

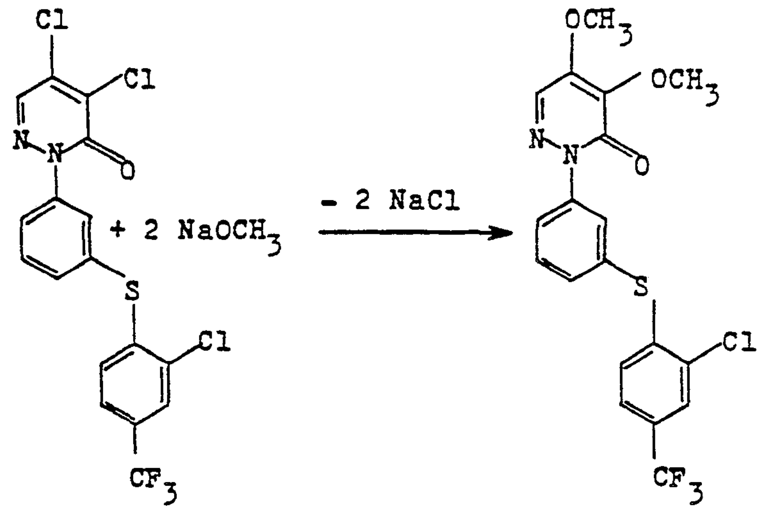


Zweckmäßigerweise wird das Dihalogenpyridazon II zunächst in einem organischen Lösungsmittel, beispielsweise in Toluol, gelöst bzw. suspendiert und dann mit der entsprechenden Menge an Alkoholat umgesetzt. Die Reaktion verläuft bei atmosphärischem oder erhöhtem Druck, im allgemeinen innerhalb von 12 Stunden bei einer Reaktionstemperatur von bis zu 100 °C, vorzugsweise bei 40 bis 80 °C ; die Umsetzung kann absatzweise oder fortlaufend vorgenommen werden. Das Reaktionsgemisch wird wie üblich aufgearbeitet. Fällt das Endprodukt fest an, so isoliert man es beispielsweise durch Absaugen des Niederschlags. Ist das Endprodukt dagegen im Lösungsmittel gelöst, wird dieses unter vermindertem Druck abdestilliert, der Rückstand mit Wasser verrührt und abgesaugt. Zur Reinigung kann das Produkt beispielsweise umkristallisiert oder chromatographiert werden.

Als Dihalogenpyridazone der Formel II können Verbindungen verwendet werden, die beispielsweise durch Umsetzung eines Phenylhydrazins der Formel

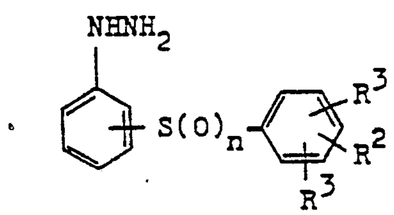

(III)

in der $R^1$, $R^2$, $R^3$ und n die obengenannten Bedeutungen besitzen, mit einer 3-Formyl-2,3-dihalogen-acrylsäure

$$HO_2C - \underset{Hal}{C} = \underset{Hal}{C} - CHO \qquad (IV)$$

in der Hal Chlor oder Brom bedeutet, erhalten werden.

Die Umsetzung zu dem entsprechenden Dihalogensäuresemicarbazon wird bei Ramtemperatur z. B. in einer mineralsauren wäßrigen Lösung oder in einem wasserhaltigen oder wasserfreien, organischen Lösungsmittel, wie Ethanol, das nach Beendigung der Reaktion abgedampft wird, vorgenommen und dieses, vorzugsweise ohne es zu isolieren, durch Kochen in Eisessig oder Essigsäureanhydrid oder durch Erwärmen in wäßriger Mineralsäure, z. B. Salzsäure, bei einer ausreichenden Temperatur von bis zu 100 °C oder durch Rühren in konzentrierter Mineralsäure, z. B. Schwefelsäure, bei Raumtemperatur zu der entsprechenden Verbindung II cyclisiert (DE-OS 16 95 840, DE-OS 25 26 643, DE-OS 15 45 595). Die Umsetzung kann man absatzweise oder fortlaufend vornehmen und das erhaltene Reaktionsprodukt nach allgemein üblichen Methoden aufarbeiten.

Die benötigten Phenylhydrazine der Formel III können ihrerseits ebenfalls nach allgemein bekannten Methoden aus entsprechenden Anilinen durch Diazotierung und Reduktion erhalten werden (Houben-Weyl, Methoden der organischen Chemie, Band 10/2, S. 180, Georg-Thieme-Verlag, Stuttgart, 1967). Die Umsetzung zum entsprechenden Pyrazon kann ohne Isolierung der Hydrazine geschehen, jedoch erhält man reinere Produkte, wenn man zuvor die Phenylhydrazine als Hydrochloride isoliert.

Die Aniline wiederum, soweit es sich um 4-Phenylthio-substituierte Aniline handelt, kann man nach den in der DE-OS 21 20 708 beschriebenen Verfahren erhalten, während die 3-Phenylthio-substituierten Aniline nach der in der EP-OS 29 123 genannten Methode zugänglich sind.

Die Oxidation von Thioethern zu entsprechenden Sulfoxiden bzw. Sulfonen ist allgemein bekannt und nicht erläuterungsbedürftig.

## Beispiel 1

a) Eine Suspension von 30,4 g 3-(2'-Chlor-4'-trifluormethylphenylthio)anilin in 200 ml Eisessig und 25 ml konz. Salzsäure wird mit einer Lösung von 6,9 g Natriumnitrit in 17,5 g Wasser bei 10 bis 20 °C umgesetzt. Dann setzt man eine Lösung von 45 g $SnCl_2$ in 31 ml 37 %iger wäßriger Salzsäure zu, wobei das entsprechende Hydrazin gebildet wird. Nach Zugabe von 16,7 g Mucochlorsäure wird die das Hydrazin enthaltende Lösung 10 Minuten bei Siedetemperatur gerührt, abgekühlt, und 1 000 ml Wasser werden hinzugefügt. Der ölige Rückstand wird in Methylenchlorid gelöst, mit Magnesiumsulfat getrocknet, unter vermindertem Druck eingeengt und der Rückstand über Kieselgel (Laufmittel Toluol/Aceton 70 : 30) chromatographiert. Man erhält 38,9 g (86 % d. Th.) 1-[3'-Chlor-4"-trifluormethylphenylthio)-phenyl]-4,5-dichlor-pyridaz-6-on mit dem Brechungsindex $n_D^{25}$ = 1,605 3.

b) Eine Suspension von 20 g 1-[3'-(2"-Chlor-4"-trifluormethylphenylthio)-phenyl]-4,5-dichlor-pyridaz-6-on 5,3 g Natriummethylat und 0,1 g N-Methylpyrrolidon in 150 ml absolutem Toluol wird zwei Stunden unter Rühren bei 60 °C gehalten. Die entstandene Lösung wird mit 200 ml Ether verdünnt, zweimal mit je 100 ml Wasser behandelt, getrocknet, filtriert und unter vermindertem Druck eingeengt. Der ölige Rückstand wird mit Diisopropylether verrieben. Man erhält 15,7 g (80 % d. Th) 1-[3'-(2"-Chlor-4"-trifluormethylphenylthio)-phenyl]-4,5-dimethoxypyridaz-6-on (Schmelzpunkt 86 bis 90 °C) durch Absaugen.

## Beispiel 2

Eine Lösung von 15 g des in dem Versuch gemäß Beispiel 1 erhaltenen Pyridazons und 7 g meta-Chlorperbenzoesäure (85 %ig) in 200 ml Chloroform werden 24 Stunden bei Raumtemperatur gerührt. Danach wird zweimal mit 100 ml gesättigter wäßriger Natriumhydrogencarbonatlösung extrahiert, getrocknet, filtriert und unter vermindertem Druck zur Trockene gebracht. Man erhält 14,9 g (96 % d. Th.) 1-[3'-(2"-Chlor-4"-trifluormethylphenylsulfinyl)-phenyl]-4,5-dimethoxy-pyridaz-6-on vom Schmelzpunkt 126 bis 130 °C).

Nach der Rahmenvorschrift des vorstehenden Beispiels 1 bzw. 2 können die in der folgenden Tabelle 1 angeführten Verbindungen hergestellt werden.

(Siehe Tabelle Seite 5 ff.)

| Beispiel Nr. | Stellung S | n | 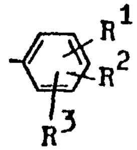 | Fp [°C], $n_D^{25}$ Wellenlänge einer Bande im IR-Spektrum |
|---|---|---|---|---|
| 3 | 3 | 2 | 2-Chlor-4-trifluormethylphenyl | $SO_2$ = 1320 $cm^{-1}$ |
| 4 | 4 | 0 | " | |
| 5 | 4 | 1 | " | |
| 6 | 4 | 2 | " | |
| 7 | 3 | 0 | 3-Chlor-4-trifluormethylphenyl | 85- 92 |
| 8 | 3 | 1 | " | |
| 9 | 3 | 2 | " | |
| 10 | 4 | 0 | " | 1,6173 |
| 11 | 4 | 1 | " | |
| 12 | 4 | 2 | " | |
| 13 | 3 | 0 | 4-Chlor-3-trifluormethylphenyl | |
| 14 | 3 | 1 | " | |
| 15 | 3 | 2 | " | |
| 16 | 4 | 0 | " | |
| 17 | 4 | 1 | " | |
| 18 | 4 | 2 | " | |
| 19 | 3 | 0 | 4-Trifluormethylphenyl | |

| Beispiel Nr. | Stellung S | n | 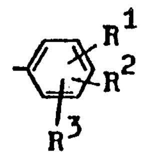 | Fp [°C], $n_D^{25}$ Wellenlänge einer Bande im IR-Spektrum |
|---|---|---|---|---|
| 20 | 4 | 0 | 3-Trifluormethylphenyl | 50- 55 |
| 21 | 4 | 1 | " | |
| 22 | 4 | 2 | 3-Trifluormethylphenyl | |
| 23 | 3 | 0 | 2-Brom-4-trifluormethylphenyl | |
| 24 | 3 | 1 | " | |
| 25 | 3 | 2 | " | |
| 26 | 3 | 0 | 2,6-Dichlor-4-trifluormethylphenyl | |
| 27 | 3 | 1 | " | |
| 28 | 3 | 2 | " | |
| 29 | 4 | 0 | 3-Chlorphenyl | 85- 90 |
| 30 | 4 | 0 | 3-Fluorphenyl | |
| 31 | 4 | 0 | 3-Trifluormethoxyphenyl | |
| 32 | 4 | 0 | 3-tert.-Butylphenyl | |
| 33 | 4 | 0 | 3-Methylphenyl | |
| 34 | 4 | 0 | 3,4-Dichlorphenyl | |
| 35 | 4 | 0 | 4-Chlorphenyl | 112-115 |
| 36 | 4 | 0 | 2,5-Dichlorphenyl | 68- 73 |
| 37 | 4 | 0 | 2,4,5-Trichlorphenyl | 120-125 |
| 38 | 4 | 0 | Phenyl | 102-105 |

Die 4,5-Dimethoxy-pyridazone der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Gießen, Stäuben, Streuen oder Sprühen auf die Pflanzen oder auf den Boden oder durch Einbringen in das Bewässerungswasser angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphtalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 10 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 7 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 29 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer

Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 20 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 2 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Anwendung der 4,5-Dimethoxy-pyradazone der Formel I bzw. der sie enthaltenden herbiziden Mittel kann im Vorauflauf- oder im Nachauflaufverfahren erfolgen. Vorzugsweise werden die Wirkstoffe bzw. die sie enthaltenden Mittel nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe geeigneter Geräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen betragen je nach Zielpflanzen, Wachstumsstadium und Witterungsverhältnissen 0,05 bis 10 kg/ha und mehr, vorzugsweise 0,1 bis 4,0 kg Wirkstoff/ha.

Der Einfluß der 4,5-Dimethoxypyridazone der Formel I auf das Wachstum von erwünschten und unerwünschten Pflanzen wird anhand von Gewächshausversuchen gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt dabei 3,0 kg Wirkstoff/ha. Nach dem Aufbringen werden die Gefäße leicht beregnet, um Keimung und Wachstum in Gang zu bringen. Danach deckt man die Gefäße mit durchsichtigen Plastikhauben ab, bis die Pflanzen angewachsen sind. Die Abdeckung bewirkt ein gleichmäßiges Keimen der Testpflanzen, sofern dies nicht durch die Wirkstoffe beeinträchtigt wird.

Für die Nachauflaufbehandlung zieht man die Testpflanzen je nach Wuchsform bis zu einer Höhe von 3 bis 15 cm an. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variierten je nach Wirkstoff. Sie betragen im Einzelfall 0,25, 0,5 1,0 bzw. 3,0 kg Wirkstoff/ha. Eine Abdeckung unterbleibt bei der Nachauflaufbehandlung.

Für wärmeliebende Arten wird eine Temperatur von etwa 20 bis 35 °C und für solche gemäßigter Klimate 10 bis 25 °C eingehalten. Die Versuche erstrecken sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normales Wachstum und 100 kein Aufgang der Pflanzen bzw. völlige Zerstörung zumindest der oberirdischen Teile.

Bei den Freilandversuchen handelt es sich um Kleinparzellen in einem mit Sojabohnen bestellten Feld und am Standort natürlich vorkommender Verunkrautung. Die herbiziden Mittel werden hierbei im Nachauflaufverfahren mit einer auf einen Traktor montierten Parzellenspritze ausgebracht. Die Bewertung erfolgt wie bei den Gewächshausversuchen.

Bei den Gewächshaus- und Freilandversuchen werden folgende Pflanzenarten getestet :

Abutilon theophrasti (Chinesischer Hanf), Amaranthus retroflexus (zurückgekrümmter Fuchsschwanz), Amaranthus spp. (Fuchsschwanz-Arten), Arachis hypogaea (Erdnüsse), Cassia tora (Gewürzrinde), Centaurea cyanus (Kornblume), Chenopodium album (Weißer Gänsefuß), Desmodium tortuosum, Echinochloa crus-gallis (Hühnerhirse), Galinsoga parviflora (Kleinblütiges Knopfkraut), Galium aparine (Klettenlabkraut), Glycine max. (Soja), Ipomoea spp. (Prunkwindearten), Lolium multiflorum (Ital. Raygras), Polygonum spp. (Knöterichärten), Sinapis alba (Weißer Senf), Solanum nigrum (Schwarzer Nachtschatten), Triticum aestivum (Weizen), Viola spp. (Stiefmütterchen), Zea mays (Mais), Stellaria media (Vogelsternmiere), Avena fatua (Flughafer), Mercurialis annua (Bingelkraut), Oryza sativa (Reis), Sorghum bicolor (Mohrenhirse).

Bei der Vorauflaufanwendung im Gewächshaus zeigen z. B. die Verbindungen Nr. 2, 7, 10 und 20 herbizide Aktivität.

Bei Nachauflaufanwendung im Gewächshaus wirken z. B. die Verbindungen Nr. 1, 2, 7, 10, 20 und 29 herbizid. Bei der Prüfung auf selektive herbizide Wirkung besitzt außerdem die Verbindung Nr. 1 mit 0,25 kg Wirkstoff/ha eine gute herbizide Wirkung gegen unerwünschte breitblättrige Pflanzen, wobei Kulturpflanzen wie Erdnüsse, Mais und Weizen, gar nicht oder nur gering und temporär geschädigt werden. Verbindung Nr. 29 bekämpft breitblättrige Unkräuter mit 0,5 kg Wirkstoff/ha selektiv. Mit 1,0 kg/ha bekämpft Verbindung Nr. 10 Hühnerhirse in Reis und gleichzeitig zeigt die Verbindung Nr. 10

ebenso wie Verbindung Nr. 7 eine herbizide Wirkung gegen verschiedene andere unerwünschte Pflanzen mit 0,5 kg Wirkstoff/ha mit nur geringen und temporären Blattschäden and der Kulturpflanze Mais.

Im Freiland lassen sich breitblättrige Unkräuter bei Nachauflaufanwendung von 0,5 kg/ha der Verbindung Nr. 1 in Soja bekämpfen, wobei an den Kulturpflanzen lediglich temporäre Blattverbrennungen in einem Ausmaß entstehen, das in Kauf genommen werden kann.

Angesichts der Vielseitigkeit der Anwendungsmethoden (Vorauflaufanwendung, Nachauflaufblattbehandlung, Unterblattspritzung) kommen viele Kulturen als Einsatzgebiete für die erfindungsgemäßen Wirkstoffe bzw. sie enthaltende Herbizide Mittel in Betracht. Solche sind beispielsweise :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagrasand |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Urdbohne |
| Phaseolus vulgaris | Buschbohnen |
| Pennisetum glaucum | Perl- oder Rohrkolbenhirse |
| Petroselinum crispum spp. tuberosum | Wurzelpetersilie |

(Fortsetzung)

| Botanischer Name | Deutscher Name |
|---|---|
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die substituierten 4,5-Dimethoxy-pyridazone der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nicht-phytotoxische Öle und Ölkonzentrate zugesetzt werden.

**Patentansprüche**

1. Substituierte 4,5-Dimethoxy-pyridazone der Formel

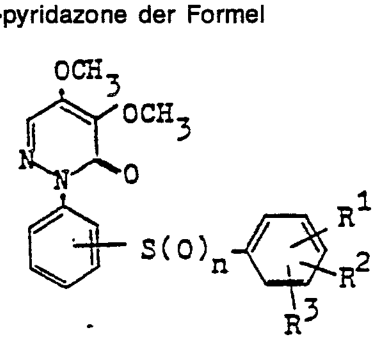

(I)

in der $R^1$, $R^2$ und $R^3$ jeweils unabhängig voneinander Wasserstoff, Halogen, Nitro, Cyano, Carboxyl,

Alkyl, Halogenalkyl, Alkoxy, Halogenalkoxy, Alkylmercapto, Halogenalkylmercapto, Alkylsulfinyl oder Alkylsulfonyl mit jeweils 1 bis 4 Kohlenstoffatomen bedeuten und n den Wert 0, 1 oder 2 hat.

2. Substituierte Pyridazone der Formel I gemäß Anspruch 1, dadurch gekennzeichnet, daß der Rest

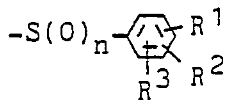

in 3- oder 4-Stellung am Phenylring und $R^1$, $R^2$ und $R^3$ in 2,4,6-, 3,4,6- oder 3,4,5-Stellung stehen, wobei $R^1$, $R^2$ und $R^3$ jeweils unabhängig voneinander Wasserstoff, Halogen oder Halogenalkyl mit 1 bis 4 Kohlenstoffatomen bedeuten.

3. 1-[3'-(2''-Chlor-4''-trifluormethyl-phenylthio)-phenyl]-4,5-dimethoxy-pyridaz-6-on.

4. 1-[3'-(2''-Chlor-4''-trifluormethyl-phenylsulfinyl)-phenyl]-4,5-dimethoxy-pyridaz-6-on.

5. Verfahren zur Herstellung substituierter 4,5-Dimethoxy-pyridazone gemäß Anspruch 1, dadurch gekennzeichnet, daß man ein Dihalogenpyridazon der Formel

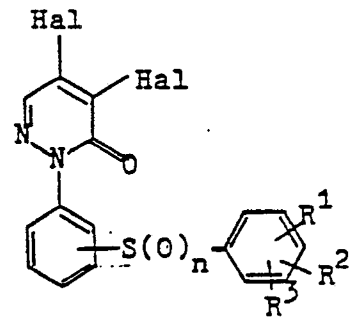

(II)

in der Hal Chlor oder Brom bedeutet, in an sich bekannter Weise mit einem Alkalimethanolat umsetzt.

6. Verfahren gemäß Anspruch 5, dadurch gekennzeichnet, daß man ein Dihalogenpyridazon der Formel II verwendet, das durch Umsetzung eines Phenylhydrazins der Formel

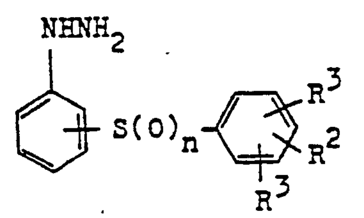

(III)

mit einer 3-Formyl-2,3-dihalogen-acrylsäure der Formel

$$HO_2C - \underset{Hal}{C} = \underset{Hal}{C} - CHO \qquad (IV)$$

in der Hal Chlor oder Brom bedeutet, zunächst bei Raumtemperatur in Gegenwart eines Lösungsmittels zum entsprechenden Dihalogensäuresemicarbazon und Cyclisierung dieses Semicarbazons durch Kochen in Eisessig oder Essigsäureanhydrid oder durch Erwärmen in wäßriger Mineralsäure bei einer ausreichenden Temperatur von bis zu 100 °C oder durch Rühren in konzentrierter Mineralsäure bei Raumtemperatur erhalten wurde.

7. Herbizid, enthaltend ein substituiertes 4,5-Dimethoxypyridazon der Formel I gemäß Anspruch 1.

8. Herbizid, enthaltend inerte Zusatzstoffe und ein substituiertes 4,5-Dimethoxy-pyridazon der Formel I gemäß Anspruch 1.

9. Verfahren zur Bekämpfung unerwünschten Pflanzenwuchses, dadurch gekennzeichnet, daß man die unerwünschten Pflanzen oder von unerwünschtem Pflanzenwachstum freizuhaltenden Flächen mit einer herbizid wirksamen Menge eines substituierten 4,5-Dimethoxy-pyridazons der Formel I gemäß Anspruch 1 behandelt.

**Claims**

1. A substituted 4,5-dimethoxypyridazone of the formula

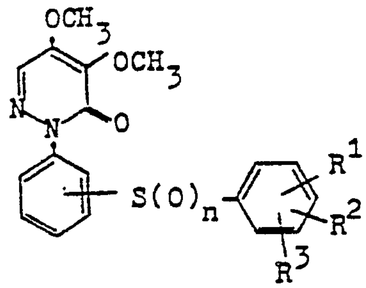

(I)

where $R^1$, $R^2$ and $R^3$ independently of one another are hydrogen, halogen, nitro, cyano, carboxyl, alkyl, haloalkyl, alkoxy, haloalkoxy, alkylmercapto, haloalkylmercapto, alkylsulfinyl or alkylsulfonyl, each of 1 to 4 carbon atoms, and n is 0, 1 or 2.

2. A substituted pyridazone of the formula I as claimed in claim 1, where

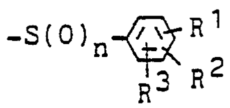

is in the 3- or 4-position on the phenyl ring, and $R^1$, $R^2$ and $R^3$ are in the 2,4,6-, 3,4,6- or 3,4,5-positions and, independently of one another, are hydrogen, halogen or haloalkyl of 1 to 4 carbon atoms.

3. 1-[3'-(2"-Chloro-4"-trifluoromethylphenylthio)-phenyl]-4,5-dimethoxypyridaz-6-one.

4. 1-[3-(2"-Chloro-4"-trifluormethylphenylsulfynyl)-phenyl]-4,5-dimethoxypyridaz-6-one.

5. A process for the preparation of a substituted 4,5-dimethoxypyridazone as claimed in claim 1, wherein a dihalopyridazone of the formula

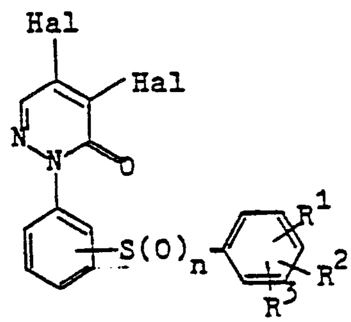

(II)

where Hal is chlorine or bromine, is reacted with an alkali metal methylate in a conventional manner.

6. A process as claimed in claim 5, wherein a dihalopyridazone of the formula II is used which has been obtained by first of all reacting a phenylhydrazine of the formula

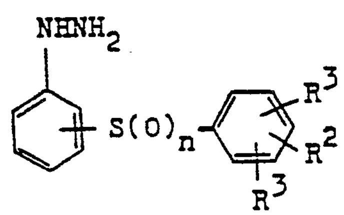

(III)

with a 3-formyl-2,3-dihaloacrylic acid of the formula

$$HO_2C - \underset{Hal}{C} = \underset{Hal}{C} - CHO \qquad (IV)$$

where Hal is chlorine or bromine, at room temperature in the presence of a solvent to give the corresponding dihalo-acid semicarbazone, and then cyclizing this semicarbazone by boiling in glacial acetic acid or acetic anhydride, or by heating in an aqueous mineral acid at a sufficiently high temperature of up to 100 °C, or by stirring in a concentrated mineral acid at room temperature.

7. A herbicide containing a substituted 4,5-dimethoxy-pyridaz-6-one of the formula I as claimed in claim 1.

8. A herbicide containing inert additives and a substituted 4,5-dimethoxypyridaz-6-one of the formula I as claimed in claim 1.

9. A process for combating unwanted plant growth, wherein the unwanted plants or the areas to be kept free from unwanted plant growth are treated with a herbicidally effective amount of a substituted 4,5-dimethoxypyridazone of the formula I as claimed in claim 1.

**Revendications**

1. 4,5-diméthoxy-pyridazones substituées de formule

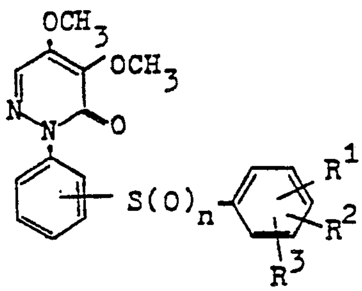

(I)

dans laquelle $R^1$, $R^2$ et $R^3$ représentent, chacun indépendamment l'un de l'autre, hydrogène, nitro, cyano, carboxyle, alkyle, halogénalkyle, alcoxy, halogénalkoxy, alkylmercapto, halogénalkylmercapto, alkylsulfinyle ou alkylsulfonyle, chacun ayant 1 à 4 atomes de carbone et n la valeur 0, 1 ou 2.

2. Pyridazones substituées de formule I, selon la revendication 1, caractérisées par le fait que le reste

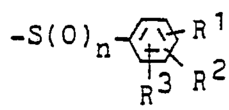

se trouve en position 3- ou 4- sur le noyau phényle et $R^1$, $R^2$ et $R^3$ se trouvent en positions 2,4,6-, 3,4,6- ou 3,4,5-, $R^1$, $R^2$ et $R^3$ représentant, chacun, indépendamment l'un de l'autre, hydrogène, halogène ou halogénalkyle à 1 à 4 atomes de carbone.

3. 1-[3'-(2"-chloro-4"-trifluorométhyl-phénylthio)-phényl]-4,5-diméthoxy-pyridaz-6-one.

4. 1-[3'-(2"-chloro-4"-trifluorométhyl-phénylsulfinyl)-phényl]-4,5-diméthoxy-pyridaz-6-one.

5. Procédé de préparation de 4,5-diméthoxy-pyridazones substituées selon la revendication 1, caractérisé par le fait qu'on fait réagir une dihalogénopyridazone de formule

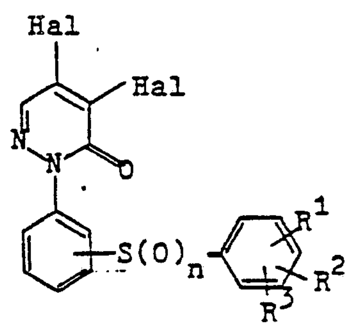

(II)

dans laquelle Hal représente chlore ou brome, de manière connue en soi, avec un méthanolate alcalin.

6. Procédé selon la revendication 5, caractérisé par le fait que l'on utilise une dihalogénopyridazone de formule II qui a été obtenue par réaction d'une phénylhydrazine de formule

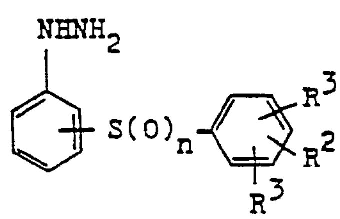

(III)

avec un acide 3-formyl-2,3-dihalogénoacrylique de formule

$$HO_2C - \underset{\displaystyle Hal}{C} = \underset{\displaystyle Hal}{C} - CHO \qquad (IV)$$

dans laquelle Hal représente chlore ou brome, d'abord à température ambiante, en présence d'un solvant, pour l'obtention de semi-carbazone dihalogénée et cyclisation de cette semi-carbazone par cuisson dans l'acide acétique cristallisable ou l'anhydride acétique, ou par chauffage en acide minéral aqueux, à une température suffisante allant jusqu'à 100 °C, ou par agitation en acide minéral concentré à la température ambiante.

7. Herbicide, contenant une 4,5-diméthoxy-pyridazone substituée de formule I selon la revendication 1.

8. Herbicide contenant des additifs inertes et une 4,5-diméthoxy-pyridazone substituée de formule I selon la revendication 1.

9. Procédé de lutte contre une croissance des plantes indésirables, caractérisé par le fait que l'on traite les plantes indésirables ou les surfaces à maintenir à l'abri d'une croissance indésirable des plantes avec une quantité efficace au point de vue herbicide, d'une 4,5-diméthoxy-pyridazone substituée de formule I selon la revendication 1.